# EUROPÄISCHE PATENTANMELDUNG

(11) **EP 3 219 783 A1**
(43) Veröffentlichungstag der Anmeldung: **20.09.2017**
(21) Anmeldenummer: 17154916.5
(22) Anmeldetag: 07.02.2017
(51) Int. Cl.: C12M 1/107, C12M 1/06, C12M 1/00, C12P 5/02

(54) **ANLAGE UND VERFAHREN ZUR VERWERTUNG VON BIOMATERIAL**

(30) Priorität: 16.03.2016 DE 102016003256
(71) Anmelder: Eisenmann SE, 71032 Böblingen (DE)
(72) Erfinder: Schlüter, Thomas, 71093 Weil im Schönbuch (DE)
(74) Vertreter: Ostertag & Partner Patentanwälte mbB

(57) **Zusammenfassung**

Die vorliegende Erfindung betrifft eine Anlage (2) zur Verwertung von Biomaterial, mit einer Gärstufe (4), wobei die Gärstufe (4) eine Fermentierkammer (10) zur Erzeugung von Biogas durch anaerobe Fermentation des Biomaterials aufweist, und mit einer Hygienestufe (6), welche der Gärstufe (4) nachgeordnet ist, wobei die Hygienestufe (6) eine Hygienisierungskammer (40, 40a, 40b) zur Aufnahme und zur thermischen Hygienisierung von aus der Gärstufe (4) ausgetragenem Biomaterial aufweist. Die Erfindung betrifft auch ein Verfahren zur Verwertung von Biomaterial durch Gärung, wobei in einer Gärstufe (4) durch anaerobe Fermentation des Biomaterials Biogas erzeugt wird, wobei das Biomaterial nach Durchlaufen der Gärstufe (4) einer Hygienestufe (6) zugeführt wird, in welcher das Biomaterial thermisch hygienisiert wird, und wobei das Biomaterial nach Durchlaufen der Hygienestufe (6) als landwirtschaftlich verwertbarer, hygienisierter Gärrest bereitgestellt wird. Derart können auch Bioabfälle, die durch hohe Störstofffrachten und Inhomogenität gekennzeichnet sind, mesophil vergoren und nach der Hygienisierung einer weiteren Nutzung, z.B. als Dünger, zugeführt werden.

## Beschreibung

Die Erfindung betrifft eine Anlage zur Verwertung von Biomaterial, mit einer Gärstufe, wobei die Gärstufe eine Fermentierkammer zur Erzeugung von Biogas durch anaerobe Fermentation des Biomaterials aufweist.

Die Erfindung betrifft auch ein Verfahren zur Verwertung von Biomaterial durch Gärung, wobei in einer Gärstufe Biogas durch anaerobe Fermentation des Biomaterials erzeugt wird.

Die DE 195 16 378 A1 beschreibt ein Verfahren zur Vergärung von organischen Reststoffen mit einem thermophilen Biogasreaktor. Da die thermophile Vergärung von stark stickstoffhaltigen Abfällen äußerst schwierig ist, werden hierbei in der Regel Verdünnungswasser und/oder Verdünnungssubstrate eingesetzt, was einen hohen verfahrenstechnischen Aufwand bedingt.

Aus der DE 197 11 355 A1 ist ein Verfahren zur Behandlung von Bioabfällen bekannt, bei dem Bioabfälle zerkleinert, gemischt und mit Wasser in Form einer Suspension nach Absiebung von Störstoffen einer Hydrolyse unterzogen werden, wobei nach der Hydrolyse und vor einer Fermentation eine Hygienisierung der Bioabfälle durchgeführt wird. Weiterhin wird gemäß der DE 197 11 355 A1 ein nach der Fermentation verbleibender Gärschlamm entwässert und einer Kompostierung zugeführt. Bei der Kompostierung kann jedoch insbesondere das Fehlen von Kohlenstoff und Strukturmaterial zu Schwierigkeiten führen.

Nachteile der bekannten Verfahren können beispielsweise der verhältnismäßig hohe apparative bzw. verfahrenstechnische Aufwand sein, sowie der relativ geringe Verwertungsgrad, der bei der Behandlung des Biomaterials erzielt wird.

Aufgabe der vorliegenden Erfindung ist es, eine Anlage zur Verwertung von Biomaterial bereitzustellen, welche eine besonders wirtschaftliche Verwertung von Biomaterial und eine gegenüber dem Stand der Technik verbesserte Verfahrensführung ermöglicht.

Diese Aufgabe wird gelöst durch eine Anlage der eingangs genannten Art, wobei die Anlage eine Hygienestufe aufweist, welche der Gärstufe nachgeordnet ist, und wobei die Hygienestufe eine Hygienisierungskammer zur Aufnahme und zur thermischen Hygienisierung von aus der Gärstufe ausgetragenem Biomaterial aufweist. Derart kann eine zuverlässige Hygienisierung auch bei verhältnismäßig niedrigen Gärtemperaturen innerhalb eines durchgehenden Verfahrens sichergestellt werden. Erfindungsgemäß wird eine wirtschaftlich vorteilhafte Verwertung und eine effiziente Hygienisierung von Biomaterial ermöglicht, auch dann, wenn sich das Biomaterial durch hohe Störstofffrachten und Inhomogenität auszeichnet. Eine nachgeschaltete Auftrennung des Gärrestes in eine flüssige und in eine feste Phase mit anschließender Hygienisierung, z. B. durch Kompostieren der Festphase und Erhitzen der Flüssigphase, kann entfallen.

Es kann vorteilhaft sein, wenn die Fermentierkammer als Reaktor mit Propfenströmung ausgebildet ist. Derart kann eine höhere Belastbarkeit sowie eine geringe Rezirkulation in der Fermentierkammer bewirkt werden. Des Weiteren ergeben sich dadurch positive Effekte auf den Abbaugrad und die Hygienisierbarkeit des Biomaterials.

Eine technisch besonders günstige Lösung ist gegeben, wenn die Hygienisierungskammer als Reaktor mit Propfenströmung ausgebildet ist. Derart können beispielsweise eine höhere Belastbarkeit und eine geringe Rezirkulation in der Hygienestufe bewirkt werden.

Es kann zweckmäßig sein, wenn das Volumen der Hygienisierungskammer kleiner ist als das Volumen der Fermentierkammer.

Eine im Hinblick auf Wirtschaftlichkeit und Verfahrensführung besonders günstige Ausgestaltung liegt vor, wenn die Fermentierkammer eine Austragseinrichtung aufweist, welche mit einer Zuführeinrichtung der Hygienisierungskammer verbunden ist.

Vorzugsweise kann eine Pumpvorrichtung zum Fördern von Biomaterial von der Austragseinrichtung der Fermentierkammer zur Zuführeinrichtung der Hygienisierungskammer vorgesehen sein.

Mit Vorteil kann die Hygienestufe mindestens zwei Hygienisierungskammern aufweisen. Derart kann eine kontinuierliche bzw. quasikontinuierliche Prozessführung begünstigt werden, wenn beispielsweise die Verweilzeit des Biomaterials in der Gärstufe länger ist als in der Hygienestufe.

Um die Prozessführung zu verbessern, kann es weiterhin von Vorteil sein, wenn die Hygienestufe derart ausgestaltet ist, dass die mindestens zwei Hygienisierungskammern parallel betrieben werden können.

Des Weiteren kann es zweckmäßig sein, wenn die Gärstufe und die Hygienestufe in einem gemeinsamen Bauwerk angeordnet sind.

Die Aufgabe der Erfindung wird auch gelöst durch ein Verfahren der eingangs genannten Art, bei dem das Biomaterial nach Durchlaufen der Gärstufe einer Hygienestufe zugeführt wird, in welcher das Biomaterial thermisch hygienisiert wird, wobei das Biomaterial nach Durchlaufen der Hygienestufe als landwirtschaftlich verwertbarer Gärrest bereitgestellt wird. Wesentliche Vorteile des Verfahrens ergeben sich in Analogie zu den Vorteilen der erfindungsgemäßen Anlage.

Vorzugsweise kann die Fermentation des Biomaterials in der Gärstufe in einem mesophilen Temperaturbereich erfolgen. Derart kann beispielsweise Biomaterial mit einem erhöhten Eiweiß- und/oder Stickstoffgehalt zuverlässig vergoren werden.

Es kann zweckmäßig sein, wenn das Biomaterial eine längere Zeit in der Gärstufe als in der Hygienestufe oder eine gleiche Zeit in der Gärstufe wie in der Hygienestufe verweilt.

Mit Vorteil kann das Biomaterial die Gärstufe in einer Propfenströmung durchlaufen. Derart können eine höhere Belastbarkeit und eine geringe Rezirkulation in der Gärstufe bewirkt werden. Des Weiteren können sich positive Effekte hinsichtlich des Abbaugrads und der Hygienisierbarkeit des Biomaterials einstellen.

In vorteilhafter Weiterbildung kann das Biomaterial die Hygienestufe in einer Propfenströmung durchlaufen. Derart können eine höhere Belastbarkeit sowie eine geringe Rezirkulation in der Hygienestufe bewirkt werden und die Effizienz der Hygienisierung kann gesteigert werden.

Vorzugsweise kann das Biomaterial in Form von Bioabfall zugeführt werden.

Beispielsweise im Hinblick auf eine kontinuierliche bzw. quasikontinuierliche Verfahrensführung kann es von Vorteil sein, wenn das Biomaterial nach Durchlaufen der Gärstufe in mindestens zwei Teilströme aufgeteilt wird, welche der Hygienestufe zugeführt werden.

Weitere vorteilhafte Ausgestaltungen der Erfindung ergeben sich aus der nachfolgenden Beschreibung. Dabei werden Ausführungsbeispiele der Erfindung, ohne hierauf beschränkt zu sein, anhand der Zeichnung näher erläutert. Es zeigen in vereinfachter, schematischer Darstellung:
- Figur 1: eine Teilansicht einer Anlage zur Verwertung von Biomaterial;
- Figur 2: eine Teilansicht einer Anlage zur Verwertung von Biomaterial in einer alternativen Ausgestaltung;
- Figur 3: eine Teilansicht einer Anlage zur Verwertung von Biomaterial in einer weiteren alternativen Ausgestaltung.

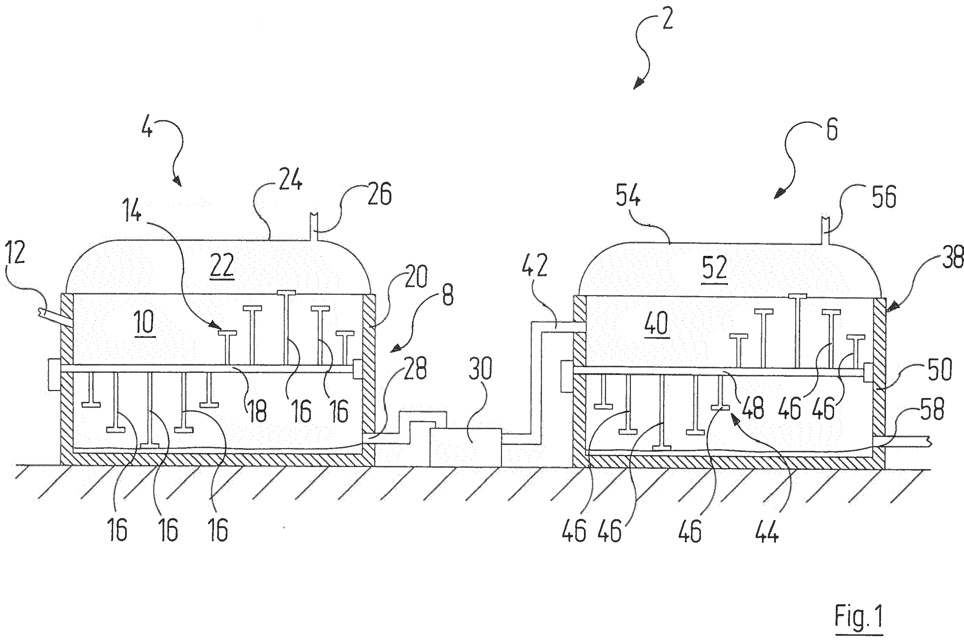

Fig. 1 zeigt einen Teil einer Anlage 2 zur Verwertung von Biomaterial mit einer Gärstufe 4 und mit einer Hygienestufe 6.

Die Gärstufe 4 ist mit einem Behälter 8 ausgestattet, der auch als Fermenterbehälter bezeichnet wird und der eine Fermentierkammer 10 zur Erzeugung von Biogas durch anaerobe Fermentation des Biomaterials aufweist. Der Behälter 8 der Gärstufe 4 weist eine Zuführeinrichtung 12 auf, die zum Eintragen von Biomaterial in die Fermentierkammer 10 ausgebildet ist. Biomaterial, welches mit Hilfe der Zuführeinrichtung 12 der Fermentation zugeführt wird, kann insbesondere Bioabfall sein, beispielsweise aus der Bioabfall-Trennung von Haushalten und/oder Reststoffe aus der Lebensmittelindustrie bzw. aus der Agrarindustrie und/oder Marktabfälle.

Im Fermenterbehälter wird energetisch verwertbares Biogas gewonnen, wobei der beispielhaft gezeigte Behälter 8 liegend angeordnet ist. Grundsätzlich können Fermenterbehälter unterschiedlichen geometrischen Aufbaus zum Einsatz kommen, welche beispielsweise liegend oder stehend angeordnet sein können. Als Materialen zur Fertigung eines Fermenterbehälters können beispielsweise Beton und/oder Stahl, insbesondere Edelstahl, verwendet werden. Stehende Fermenterbehälter können beispielsweise mit einem runden Querschnitt ausgeführt sein. Liegende Fermenterbehälter können beispielsweise einen rechteckigen oder quadratischen Querschnitt aufweisen. Liegende Fermenterbehälter können beispielsweise auch im Wesentlichen zylindrisch ausgebildet sein.

Der in Fig. 1 schematisch dargestellte Behälter 8 der Gärstufe 4 kann z.B. als sogenannter Pfropfenstromfermenter ausgebildet sein, wobei die Fermentierkammer 10 einen Reaktor mit Propfenströmung ausbildet. Dabei wird der Inhalt des Behälters 8 auf Grund des Verdrängungseffekts des eingebrachten Materials - ähnlich einem Pfropfen - in horizontaler Richtung durch die Fermentierkammer 10 transportiert. Das Biomaterial durchläuft die Fermentierkammer 10 in einer Propfenströmung. Die als Reaktor mit Propfenströmung ausgebildete Fermentierkammer 10 kann beispielsweise kontinuierlich oder quasikontinuierlich mit Biomaterial beschickt werden.

In der Fermentierkammer 10 ist ein Rührwerk 14 vorgesehen. Das Rührwerk 14 weist eine Rührwelle 18 auf, an der nur teilweise mit Bezugszeichen versehene Rührwerkzeuge 16 angeordnet sind. Mittels des Rührwerks 14 erfolgt eine Durchmischung des Fermenterinhalts, wobei die Bildung von Schwimm- und Sinkschichten verringert wird und die mikrobiologische Aktivität im Fermenter positiv beeinflusst wird. Im gezeigten Beispiel ist das Rührwerk 14 als Haspelrührwerk ausgebildet, wobei die Rührwerkzeuge 16 als Rührarme entlang der Rührwelle 18 angeordnet sind. Im gezeigten Beispiel ragen die Rührwerkzeuge 16 von der Rührwelle 18 radial nach außen. Es kann vorteilhaft sein, wenn die Rührwerkzeuge 16 zumindest in einem Teilbereich des Behälters 8 spiralförmig angeordnet sind und - wie in der Zeichnung angedeutet - an ihren Enden mit Paddeln ausgestattet sind. Beim vorliegenden Ausführungsbeispiel verlaufen diese Paddel parallel zur Rührwelle 18.

Das Biogas, welches durch die Fermentation des Biomaterials in dem Behälter 8 der Gärstufe 4 entsteht, sammelt sich in einem Gasspeicher 22 unterhalb einer Abdeckung 24. Wie im Beispiel schematisch angedeutet, wird der Gasspeicher 22 nach oben durch die Abdeckung 24 und nach unten durch den Spiegel des in der Fermentierkammer 10 befindlichen Materials begrenzt. Die Abdeckung 24 kann beispielsweise als Folienhaube aus flexiblem, gasdichten Material ausgebildet sein. Eine andere Ausführungsform der Abdeckung 24 ist beispielsweise ein sogenanntes Tragluftdach. Bei einem Tragluftdach ist in der Regel eine Außenmembrane vorgesehen unterhalb derer eine Gasspeichermembrane angeordnet ist, durch welche der Gasspeicher nach oben begrenzt wird. Alternativ oder zusätzlich zur gezeigten Anordnung des Gasspeichers 22 ist es auch möglich einen gesonderten Speicher zur Gaslagerung vorzusehen. Ein gesonderter Speicher kann z.B. ein externer Foliengasspeicher sein.

Mittels einer in der Zeichnung lediglich angedeuteten Gasentnahmeeinrichtung 26 kann dem Gasspeicher 22 das im Fermenterbehälter gewonnene Biogas entnommen werden und einer Nutzung und/oder einer weiteren Aufbereitung und/oder einer Lagerung zugeführt werden.

Im gezeigten Beispiel ist auf der Seite des Gehäuses 20 des Fermenterbehälters, welche der Zuführeinrichtung 12 gegenüberliegt, eine Austragseinrichtung 28 für das vergorene Biomaterial vorgesehen.

Die in Fig. 1 gezeigte Anlage zur Verwertung von Biomaterial, insbesondere Bioabfall wird derart betrieben, dass das Biomaterial nach Durchlaufen der Gärstufe 4 einer der Gärstufe 4 nachgeordneten Hygienestufe 6 zugeführt wird. Die Hygienestufe 6 ist mit einem Behälter 38 ausgestattet, in dem von der Gärstufe 4 zugeführtes Biomaterial thermisch hygienisiert wird.

Grundsätzlich kann der Behälter 38 der Hygienestufe 6 in seiner Ausführung, z.B. hinsichtlich seines Aufbaus, der verwendeten Materialien und seiner Anordnung dem vorangehend beschriebenen Fermenterbehälter ähneln. Der Behälter 38 der Hygienestufe 6 weist eine Hygienisierungskammer 40 auf, die mit einer Zuführeinrichtung 42 für Biomaterial ausgestattet ist. Im gezeigten Beispiel ist auf der Seite des Gehäuses 50 dieses Behälters 38, welche der Zuführeinrichtung 42 gegenüberliegt, eine Austragseinrichtung 58 für das hygienisierte Biomaterial vorgesehen.

Die in Fig. 1 schematisch dargestellte Hygienisierungskammer 40 kann vorzugsweise als Reaktor mit Propfenströmung ausgebildet sein. Dabei wird der Inhalt des Behälters 38 auf Grund des Verdrängungseffekts des eingebrachten Materials - ähnlich einem Pfropfen - in horizontaler Richtung durch die Hygienisierungskammer 40 transportiert. Das Biomaterial durchläuft die Hygienisierungskammer 40 in einer Propfenströmung. Die als Reaktor mit Propfenströmung ausgebildete Hygienisierungskammer 40 kann beispielsweise kontinuierlich oder quasikontinuierlich mit Biomaterial beschickt werden. Mit Vorteil kann als Material zum Ausbilden der Hygienisierungskammer 40 Stahl verwendet werden.

Im gezeigten Beispiel ist die Hygienisierungskammer 40 vergleichbar der zuvor beschriebenen Fermentierkammer 10 mit einem Rührwerk 44 ausgestattet, das eine Rührwelle 48 aufweist, an der nur teilweise mit Bezugszeichen versehene Rührwerkzeuge 46 angeordnet sind.

Auch in der Hygienisierungskammer 50 kann Biogas entstehen. Das in der Hygienisierungskammer 50 entstandene Biogas sammelt sich in einem Gasspeicher 52 unterhalb einer Abdeckung 54, wobei der Gasspeicher 52 und die Abdeckung 54 der Hygienestufe grundsätzlich ähnlich wie der Gasspeicher 22 und die Abdeckung 24 der Gärstufe 4 ausgeführt sein können. Auch in der Hygienestufe kann, wie in der Zeichnung angedeutet, eine Gasentnahmeeinrichtung 56 vorgesehen sein. Mittels einer Gasentnahmeeinrichtung 56 kann dem Gasspeicher 52 der Hygienestufe 6 Biogas entnommen werden und einer Nutzung und/oder Lagerung zugeführt werden.

Gemäß dem gezeigten Ausführungsbeispiel ist die Austragseinrichtung 28 des Behälters 8 der Gärstufe 4 mit der Zuführeinrichtung 42 des Behälters 38 der Hygienestufe 6 verbunden, um Biomaterial von der Fermentierkammer 10 zur Hygienisierungskammer 40 zu fördern. Dabei wird das Biomaterial vorzugsweise mittels einer Pumpvorrichtung 30 von der Austragseinrichtung 28 der Gärstufe 4 zur Zuführeinrichtung 42 der Hygienestufe 6 gepumpt.

Insbesondere wenn sowohl die Fermentierkammer 10 als auch die Hygienisierungskammer 40 nach dem Prinzip der Propfenströmung arbeiten, kann es zweckmäßig sein, wenn das Volumen der Hygienisierungskammer 40 kleiner ist als das Volumen der Fermentierkammer 10.

Sowohl die Gärstufe 4 als auch die Hygienestufe 6 sind zur Verwertung von Biomaterial, beispielsweise Bioabfall, mit einem hohen Gehalt von Störstoffen und Trockensubstanz ausgelegt.

Die Vergärung des Biomaterials, beispielsweise Bioabfall, in der Gärstufe 4 erfolgt im mesophilen Temperaturbereich, d.h. bei vergleichsweise niedriger Temperatur, z. B. bei ca. 40°C bzw. in einem Temperaturbereich von ca. 37°C bis ca. 40°C.

In der Hygienestufe 6, die der Gärstufe 4 nachgeordnet ist, werden Keime thermisch abgetötet und es erfolgt eine weitestgehende Entgasung des Biomaterials. Bei einer Hygienisierungstemperatur von ca. 60° und darüber werden Methanbakterien abgetötet. Die Methanemissionen ausgehend von Biomaterial, welches die Hygienestufe 6 durchlaufen hat, können derart deutlich verringert werden. Eine besonders sichere Zwischenlagerung wird somit ermöglicht.

Die Hygienestufe 6 ist für einen Betrieb bei vorzugsweise bis zu ca. 70°C ausgelegt. Derart liegt an der Austragseinrichtung 58 der Hygienestufe 6 hygienisch unbedenkliches Biomaterial vor, welches beispielsweise in der Landwirtschaft als hochwertiges Düngemittel genutzt werden kann. Dabei können die Verordnung (EG) Nr. 1069/2009 und die Verordnung (EU) Nr. 142/2011 eingehalten werden.

Der Fermenterbehälter der Gärstufe 4 ist auf die erforderliche Verweilzeit des Biomaterials bzw. des entsprechenden Substrats ausgelegt, wobei die reale Mindestverweilzeit in der Gärstufe 4 z. B. ca. 14 Stunden betragen kann. Der Behälter 38 der Hygienestufe 6 ist auf die zur Hygienisierung erforderliche Verweilzeit des Biomaterials ausgelegt, wobei die reale Mindestverweilzeit in der Hygienestufe 6 z. B. ca. 7 Stunden betragen kann. Es kann vorteilhaft sein, wenn die Verweilzeit des Biomaterials in der Hygienestufe 6 geringer ist als die Verweilzeit in der Gärstufe 4. Die Verfahrenstemperatur in der Hygienestufe 6 ist deutlich höher als die Verfahrenstemperatur in der Gärphase 4.

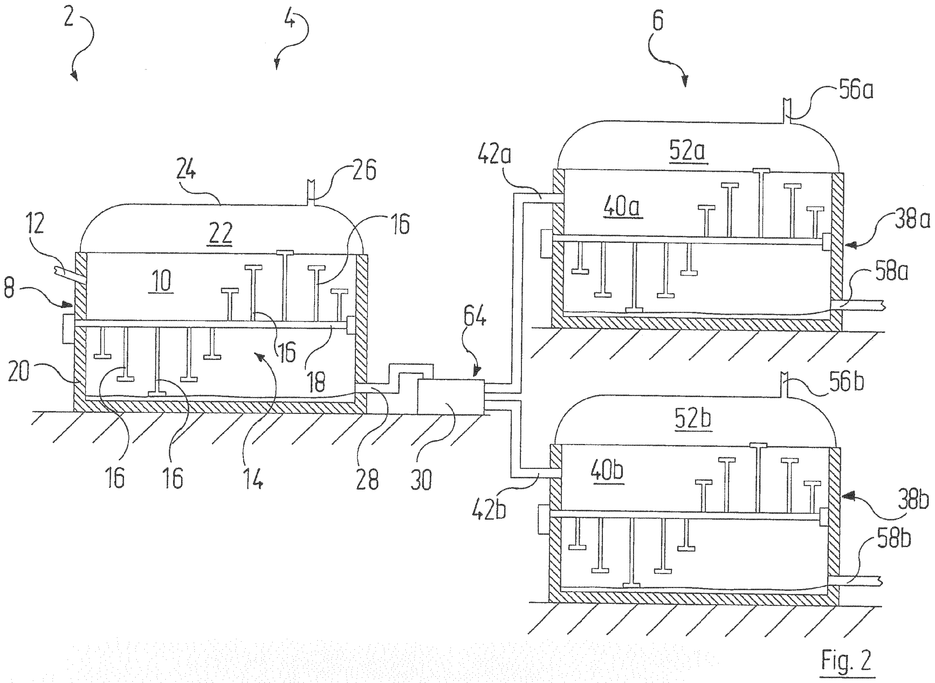

Fig. 2 zeigt ein weiteres Beispiel einer Anlage 2 zur Verwertung von Biomaterial mit einer Gärstufe 4 und mit einer Hygienestufe 6, wobei die hier gezeigte Gärstufe 4 hinsichtlich ihrer Ausführung der vorangehend im Zusammenhang mit Fig. 1 beschriebenen Gärstufe 4 entspricht.

Die Hygienestufe 6 gemäß Fig. 2 ist mit einem ersten Behälter 38a und mit einem zweiten Behälter 38b ausgestattet. Diese Behälter 38a, 38b weisen jeweils eine Hygienisierungskammer 40a bzw. 40b und einen zugeordneten Gasspeicher 52a bzw. 52b auf. Die Gasspeicher 52, 52b weisen jeweils eine Gasentnahmeeinrichtung 56a bzw. 56b auf. Die Hygienisierungskammern 40a, 40b weisen jeweils eine Zuführeinrichtung 42a bzw. 42b für Biomaterial auf. Auf der den Zuführeinrichtungen 42a, 42b gegenüberliegenden Seite der Behälter 38a, 38b sind jeweils Austragseinrichtungen 58a bzw. 58b für das hygienisierte Biomaterial vorgesehen. Weitere Einzelheiten der Behälter 38a, 38b sowie der Hygienisierungskammern 40a, 40b und der Gasspeicher 52a, 52b sind der besseren Übersicht halber in Fig. 2 nicht mit Bezugszeichen versehen.

Die erste Hygienisierungskammer 40a und die zweite Hygienisierungskammer 40b der Hygienestufe 6 sind prozesstechnisch und im Hinblick auf den Materialfluss des Biomaterials einander nebengeordnet. Die zwei Hygienisierungskammern 40a und 40b der Hygienestufe 6 sind der Fermentierkammer 10 der Gärstufe 4 nachgeordnet.

Die Austragseinrichtung 28 des Behälters 8 der Gärstufe 4 ist gemäß dem in Fig. 2 gezeigten Beispiel sowohl mit der Zuführeinrichtung 42a des ersten Behälters 38a als auch mit der Zuführeinrichtung 42b des zweiten Behälters 38b der Hygienestufe 6 verbunden. Um Biomaterial von der Fermentierkammer 10 zu den Hygienisierungskammern 40a, 40b zu fördern, ist eine Fördervorrichtung vorgesehen, die im gezeigten Beispiel eine Pumpvorrichtung 30 aufweist.

Die Fördervorrichtung weist zudem eine Teilervorrichtung 64 auf, welche den von der Fermentierkammer 10 kommenden Strom des Biomaterials in mehrere Teilströme aufteilt. Im gezeigten Beispiel führt ein erster Teilstrom von der Teilervorrichtung 64 zur Zuführeinrichtung 42a des ersten Behälters 38a der Hygienestufe 6. Ein zweiter Teilstrom führt von der Teilervorrichtung 64 zur Zuführeinrichtung 42b des zweiten Behälters 38b der Hygienestufe 6. Die beiden Hygienisierungskammern 40a, 40b werden gemäß dem hier gezeigten Ausführungsbeispiel parallel betrieben. Die Aufteilung des aus der Fermentierkammer 10 ausgetragenen Biomaterials auf mehrere Hygienisierungskammern 40a, 40b kann beispielsweise gerade dann von Vorteil sein, wenn die Anlage 2 vorzugsweise derart ausgelegt ist, dass die Verweilzeit des Biomaterials in der Hygienestufe 6, um eine zuverlässige Hygienisierung des Gärproduktes sicherzustellen. Dabei begünstigt die in Fig. 2 gezeigte Ausgestaltung der Anlage 2 einen kontinuierlichen bzw. quasikontinuierlichen Betrieb der Gärstufe 4 und der Hygienestufe 6.

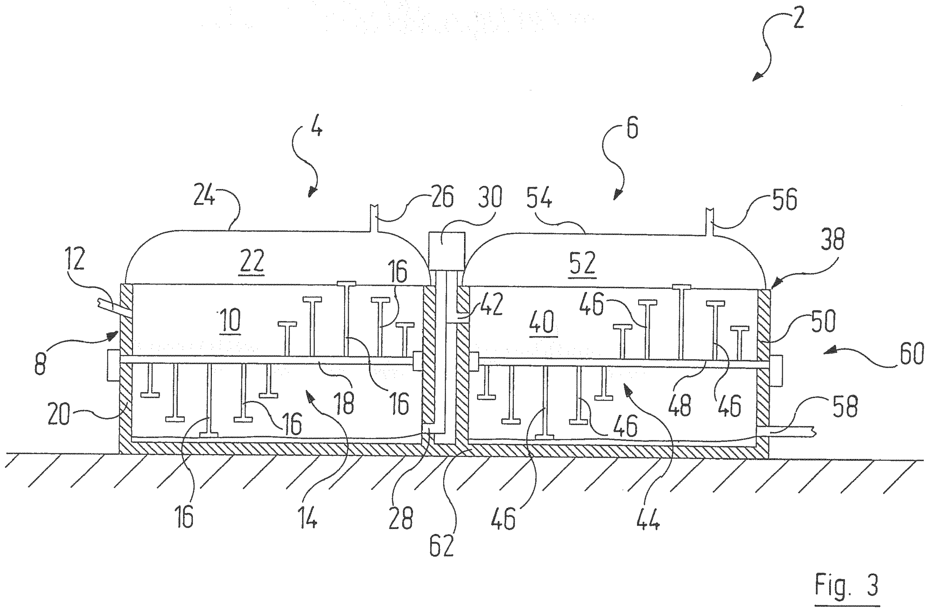

Auch Fig. 3 zeigt einen Teil einer Anlage 2 zur Verwertung von Biomaterial mit einer Gärstufe 4 und mit einer Hygienestufe 6. Die hier beispielhaft gezeigte Anlage 2 unterscheidet sich von dem in Fig. 1 gezeigten Beispiel einer Anlage2 dadurch, dass die Gärstufe 4 und die Hygienestufe 6 gemäß Fig. 3 in ihrer baulichen Ausführung vereint sind. Dabei können das Gehäuse 20 des Behälters 8 der Gärstufe 4 und das Gehäuse 50 des Behälters 38 der Hygienestufe 6 beispielsweise gemeinsame Elemente, wie z. B. eine gemeinsame Bodenplatte 62, aufweisen.

Während die Gärstufe 4 und die Hygienestufe 6 gemäß Fig. 1 als räumlich getrennte Bauwerke ausgeführt sind, können die Gärstufe 4 und die Hygienestufe 6 gemäß Fig. 3 alternativ auch in einem gemeinsamen Bauwerk ausgeführt sein.

Ein Gedanke, welcher der Erfindung zugrunde liegt, lässt sich wie folgt zusammenfassen: Die vorliegende Erfindung betrifft eine Anlage 2 zur Verwertung von Biomaterial, mit einer Gärstufe 4, wobei die Gärstufe 4 eine Fermentierkammer 10 zur Erzeugung von Biogas durch anaerobe Fermentation des Biomaterials aufweist, und mit einer Hygienestufe 6, welche der Gärstufe 4 nachgeordnet ist, wobei die Hygienestufe 6 eine Hygienisierungskammer 40, 40a, 40b zur Aufnahme und zur thermischen Hygienisierung von aus der Gärstufe 4 ausgetragenem Biomaterial aufweist. Die Erfindung betrifft auch ein Verfahren zur Verwertung von Biomaterial durch Gärung, wobei in einer Gärstufe 4 durch anaerobe Fermentation des Biomaterials Biogas erzeugt wird, wobei das Biomaterial nach Durchlaufen der Gärstufe 4 einer Hygienestufe 6 zugeführt wird, in welcher das Biomaterial thermisch hygienisiert wird, und wobei das Biomaterial nach Durchlaufen der Hygienestufe 6 als landwirtschaftlich verwertbarer, hygienisierter Gärrest bereitgestellt wird. Derart können auch Bioabfälle, die durch hohe Störstofffrachten und Inhomogenität gekennzeichnet sind, mesophil vergoren und nach der Hygienisierung einer weiteren Nutzung, z.B. als Dünger, zugeführt werden.

## Patentansprüche

1. Anlage (2) zur Verwertung von Biomaterial, mit einer Gärstufe (4), wobei
(a) die Gärstufe (4) eine Fermentierkammer (10) zur Erzeugung von Biogas durch anaerobe Fermentation des Biomaterials aufweist,
**dadurch gekennzeichnet, dass**
(b) die Anlage (2) eine Hygienestufe (6) aufweist, welche der Gärstufe (4) nachgeordnet ist, und dass
(c) die Hygienestufe (6) eine Hygienisierungskammer (40, 40a, 40b) zur Aufnahme und zur thermischen Hygienisierung von aus der Gärstufe (4) ausgetragenem Biomaterial aufweist.

2. Anlage (2) nach Anspruch 1, **dadurch gekennzeichnet, dass** die Fermentierkammer (10) als Reaktor mit Propfenströmung ausgebildet ist.

3. Anlage (2) nach Anspruch 1 oder 2, **dadurch gekennzeichnet, dass** die Hygienisierungskammer (40, 40a, 40b) als Reaktor mit Propfenströmung ausgebildet ist.

4. Anlage (2) nach einem der Ansprüche 1 bis 3, **dadurch gekennzeichnet, dass** das Volumen der Hygienisierungskammer (40, 40a, 40b) kleiner als das Volumen der Fermentierkammer (10) ist.

5. Anlage (2) nach einem der Ansprüche 1 bis 4, **dadurch gekennzeichnet, dass** die Fermentierkammer (10) eine Austragseinrichtung (28) aufweist, welche mit einer Zuführeinrichtung (42, 42a, 42b) der Hygienisierungskammer (40, 40a, 40b) verbunden ist.

6. Anlage (2) nach Anspruch 5, **dadurch gekennzeichnet, dass** eine Pumpvorrichtung (30) zum Fördern von Biomaterial von der Austragseinrichtung (28) der Fermentierkammer (10) zur Zuführeinrichtung (42, 42a, 42b) der Hygienisierungskammer (40, 40a, 40b) vorgesehen ist.

7. Anlage (2) nach einem der Ansprüche 1 bis 6, **dadurch gekennzeichnet, dass** die Hygienestufe (6) mindestens zwei Hygienisierungskammern (40a, 40b) aufweist.

8. Anlage (2) nach Anspruch 7, **dadurch gekennzeichnet, dass** die Hygienestufe (6) derart ausgestaltet ist, dass die mindestens zwei Hygienisierungskammern (40a, 40b) parallel betrieben werden können.

9. Anlage (2) nach einem der Ansprüche 1 bis 8, **dadurch gekennzeichnet, dass** die Gärstufe (4) und die Hygienestufe (6) in einem gemeinsamen Bauwerk (60) angeordnet sind.

10. Verfahren zur Verwertung von Biomaterial durch Gärung, wobei
(a) in einer Gärstufe (4) Biogas durch anaerobe Fermentation des Biomaterials erzeugt wird,
**dadurch gekennzeichnet, dass**
(b) das Biomaterial nach Durchlaufen der Gärstufe (4) einer Hygienestufe (6) zugeführt wird, in welcher das Biomaterial thermisch hygienisiert wird, und dass
(c) das Biomaterial nach Durchlaufen der Hygienestufe (6) als landwirtschaftlich verwertbarer Gärrest bereitgestellt wird.

11. Verfahren nach Anspruch 10, **dadurch gekennzeichnet, dass** die Fermentation des Biomaterials in der Gärstufe (4) in einem mesophilen Temperaturbereich erfolgt.

12. Verfahren nach Anspruch 10 oder 11, **dadurch gekennzeichnet, dass** das Biomaterial eine längere Zeit in der Gärstufe (4) als in der Hygienestufe (6) oder eine gleiche Zeit in der Gärstufe (4) wie in der Hygienestufe (6) verweilt.

13. Verfahren nach einem der Ansprüche 10 bis 12, **dadurch gekennzeichnet, dass** das Biomaterial die Gärstufe (4) in einer Propfenströmung durchläuft.

14. Verfahren nach einem der Ansprüche 10 bis 13, **dadurch gekennzeichnet, dass** das Biomaterial die Hygienestufe (6) in einer Propfenströmung durchläuft.

15. Verfahren nach einem der Ansprüche 10 bis 14, **dadurch gekennzeichnet, dass** das Biomaterial in Form von Bioabfall zugeführt wird.

16. Verfahren nach einem der Ansprüche 10 bis 15, **dadurch gekennzeichnet, dass** das Biomaterial nach Durchlaufen der Gärstufe (4) in mindestens zwei Teilströme aufgeteilt wird, welche der Hygienestufe (6) zugeführt werden.
